# EUROPEAN PATENT APPLICATION

(11) **EP 3 932 475 A1**
(43) Date of publication of application: **05.01.2022**
(21) Application number: 21182642.5
(22) Date of filing: 30.06.2021
(51) Int. Cl.: A61N 1/36, A61N 1/05

(54) **SYSTEMS, DEVICES, COMPONENTS AND METHODS FOR THE DELIVERY OF FIRST AND SECOND ELECTRICAL STIMULATION SIGNALS TO MOTOR AND SENSORY PERIPHERAL TARGET NERVES**

(30) Priority: 30.06.2020 US 202016917326
(71) Applicant: Neuro Rehab Systems, LLC, White Bear Lake MN 55110 (US)
(72) Inventor: STYLOS, Lee, Stillwater, 55082 (US); GAGNON, Jeffrey, Champlin, 55316 (US); SWOYER, John, Blaine, 55449 (US); DREES, Scott, Dallas, 75219 (US)
(74) Representative: Lecomte & Partners

(57) **Abstract**

Disclosed are various examples and embodiments of systems, devices, components and methods configured to rehabilitate or strengthen one or more muscles in a patient, and to reduce pain sensed by the patient, through a unique combination of electrical stimulation signals delivered to one or more target peripheral nerves. Medical electrical lead(s) comprising electrode(s) are positioned adjacent to, in contact with, or in operative positional relationship to, one or more target peripheral nerves of the patient. The target peripheral nerves typically comprise motor and sensory nerves. In one embodiment, first stimulation signals having a first range of frequencies are delivered through the electrode(s) to the target nerves to rehabilitate muscles enervated by the motor nerves. Second stimulation signals having a second range of frequencies are delivered through the electrode(s) to the target nerves to provide pain relief to the patient. The first range of frequencies is lower than the second range of frequencies.

## Description

### Field of the Invention

Various embodiments described and disclosed herein relate to the field of neurostimulation, and more particularly to delivering electrical stimulation therapy to peripheral nerves of a patient, including, but not limited to, for the purpose of stimulating muscles and alleviating pain.

### Background

Chronic low back pain (LBP) is the number one total cost burden to the U.S. healthcare system at approximately $600,000,000 per year according to ScienceDaily and a Johns Hopkins University health economics study published in September, 2012 in the Journal of Pain. LBP treatments can span the gamut from low cost over-the-counter pharmaceuticals and opioids all the way to costly spinal interventions. Frequently, marginal clinical results and/or unwanted drug dependencies result from these strategies.

Chronic LBP sufferers frequently have pain resulting from unidentified causes, and are referred to as Non-Specific Chronic Low Back Pain (NSCLBP) patients, of whom there are some 60 million such patients annually in the U.S. NSCLBP is a sub-category of LBP. NSCLBP is clinically determined by exclusion, and is defined as unmitigated chronic low back pain lasting longer than 120 days that is not attributable to a recognizable known specific pathology (e.g., infection, tumor, osteoporosis, lumbar spine fracture, disk deterioration, congenital or structural deformities, inflammatory disorders, radicular syndromes, nerve diseases or cauda equina syndrome).

NSCLBP patients suffer from a recurrent cycle of intense unidentified chronic low back pain and muscle atrophy, creating long-term spinal instability.

What is needed are improved means and methods of treating NSCLB patients, such as an acute, low cost, least invasive, Peripheral Nerve Stimulation (PNS) system that can provide relief, rehabilitation and restoration early in the patient treatment continuum.

The present disclosure is directed to devices, systems, and methods that address one or more deficiencies in the prior art.

### Summary

In some embodiments, there is provided a method of rehabilitating or strengthening one or more muscles in a patient, and reducing pain sensed by the patient, though electrical stimulation of one or more peripheral nerves, comprising positioning one or more medical electrical leads comprising one or more electrodes adjacent to, in contact with, or in operative positional relationship to, one or more target peripheral nerves of the patient, the one or more target peripheral nerves comprising motor and sensory nerves; delivering first stimulation signals having a first range of frequencies through the one or more electrodes of the one or more medical electrical leads to the one or more target peripheral nerves; delivering second stimulation signals having a second range of frequencies through the one or more electrodes of the one or more medical electrical leads to the one or more target nerves; wherein the first range of frequencies is lower than the second range of frequencies, the first stimulation signals are configured to stimulate one or more motor nerves in the one or more target peripheral nerves to rehabilitate or strengthen the one or more muscles, the second stimulation signals are configured to stimulate one or more sensory nerves in the one or more target peripheral nerves to reduce pain sensed by the patient.

In other embodiments, there is a provided a system for rehabilitating or strengthening one or more muscles in a patient, and reducing pain sensed by the patient, though electrical stimulation of one or more peripheral nerves, comprising one or more percutaneous medical electrical leads comprising distal portions or ends comprising one or more electrodes configured for implantation adjacent to, in contact with, or in operative positional relationship to, one or more target peripheral nerves of the patient, where the one or more bundles of target peripheral nerves comprising motor and sensory nerves, and an external pulse generator (EPG) configured for operable connection to the one or more medical electrical leads, and further being configured to deliver first stimulation signals having a first range of frequencies through the one or more electrodes of the one or more medical electrical leads to the one or more bundles of target peripheral nerves, the EPG still further being configured to deliver second stimulation signals having a second range of frequencies through the one or more electrodes of the one or more medical electrical leads to the one or more bundles of target nerves; wherein the first range of frequencies is lower than the second range of frequencies, the first stimulation signals are configured to stimulate one or more motor nerves in the one or more bundles of target peripheral nerves to disrupt arthrogenic inhibition of the one or more muscles and to rehabilitate or strengthen the one or more muscles, the second stimulation signals are configured to stimulate one or more sensory nerves in the one or more bundles of target peripheral nerves to engage gate mechanisms associated therewith thereby to reduce pain sensed by the patient.

According to a preferred embodiment, the one or more medical electrical leads are percutaneous leads.

According to a preferred embodiment, the one or more target peripheral nerves comprise bundles of nerves.

According to a preferred embodiment, the first stimulation signals are further configured to disrupt arthrogenic inhibition of the one or more muscles.

According to a preferred embodiment, the second stimulation signals are configured to engage gate mechanisms associated with the one or more sensory nerves thereby to reduce the pain sensed by the patient.

According to a preferred embodiment, one or more stimulation parameters of the first stimulation signals comprise one or more of: (a) frequencies ranging between about 2 Hz and about 100 Hz; (b) frequencies ranging between about 2 Hz and about 75 Hz; (c) frequencies ranging between about 4 Hz and about 50 Hz; (d) frequencies ranging between about 5 Hz and about 25 Hz; (e) frequencies ranging between about 7 Hz and about 100 Hz; (f) voltage ranging between about 0.1 mV and about 30 V; (g) current ranging between about 0.1 mA and about 30 mA; pulse width ranging between about 20 µsec and about 1000 µsec.

According to a preferred embodiment, one or more stimulation parameters of the second stimulation signals comprise one or more of: (a) frequencies ranging between about 100 Hz and about 10,000 Hz; (b) frequencies ranging between about 100 Hz and about 5,000 Hz; (c) frequencies ranging between about 100 Hz and about 2,000 Hz; (d) frequencies ranging between about 100 Hz and about 1,000 Hz; (e) frequencies ranging between about 200 Hz and about 750 Hz; (f) voltage ranging between about 0.1 mV and about 30 V; (g) current ranging between about 0.1 mA and about 30 mA; pulse width ranging between about 20 µsec and about 1000 µsec.

According to a preferred embodiment, the first stimulation signals are interleaved or alternate with the second stimulation signals.

According to a preferred embodiment, the first stimulation signals overlap with the second stimulation signals.

According to a preferred embodiment, the first stimulation signals are at least partially superimposed upon and delivered simultaneously with the second stimulation signals.

According to a preferred embodiment, the first stimulation signals are delivered to the one or more target nerves at different times than when the second stimulation signals are delivered to the one or more bundles of target nerves.

According to a preferred embodiment, the first stimulation signals are delivered to the one or more target nerves for periods of time ranging between about 60 seconds and about 180 minutes.

According to a preferred embodiment, the second stimulation signals are delivered to the one or more target nerves for periods of time ranging between about 60 seconds and about 180 minutes.

According to a preferred embodiment, the first stimulation signals are delivered to the one or more target nerves in bursts ranging between about 20 seconds and about 60 seconds in duration.

According to a preferred embodiment, the second stimulation signals are delivered to the one or more target nerves in bursts ranging between about 20 seconds and about 120 seconds in duration.

According to a preferred embodiment, delivery of the first stimulation signals is separated from delivery of the second stimulation signals by a period of time ranging between: (a) about 0 seconds and about 60 seconds; (b) about 2 minutes and about 120 minutes; and (c) about 1hour and about 3 hours.

According to a preferred embodiment, the one or more target peripheral nerves comprise dorsal rami nerves.

According to a preferred embodiment, the one or more electrodes are positioned proximal to a bifurcation of medial and distal branches of the dorsal rami nerves.

According to a preferred embodiment, the one or more muscles comprise one or more multifidus muscles.

According to a preferred embodiment, the first stimulation signals promote rehabilitating or strengthening one or more atrophied multifidus muscles.

According to a preferred embodiment, the pain is non-specific chronic low back pain (NSCLBP).

According to a preferred embodiment, the second stimulation signals promote reducing non-specific chronic lower back pain.

According to a preferred embodiment, the one or more target peripheral nerves are located in or near one or more of the patient's shoulder, neck, arm, leg, knee, hip, foot, or ankle.

According to a preferred embodiment, the one or more medical electrical leads comprise at least one of a unipolar electrode, a bipolar electrode, a ground electrode, a cathode, an anode, a coiled electrode, a cuff electrode, a wire electrode, and a hook-shaped electrode.

According to a preferred embodiment, ultrasound or fluoroscopy are employed to guide placement of a needle to locate the one or more target peripheral nerves.

According to a preferred embodiment, the needle is hollow and used to deliver one of the medical electrical leads to the one or more target peripheral nerves percutaneously.

According to a preferred embodiment, an MRI is used to image one or more multifidus muscles in the patient to assess the strength or degree of atrophy of the multifidus muscles before the medical electrical lead is implanted in the patient.

According to a preferred embodiment, an MRI is used to image one or more multifidus muscles in the patient after therapy has been delivered to the patient by the first and second stimulation signals and after the medical electrical lead has been implanted in the patient.

Further embodiments are disclosed herein or will become apparent to those skilled in the art after having read and understood the claims, specification and drawings hereof.

### Brief Description of the Drawings

Different aspects of the various embodiments will become apparent from the following specification, drawings and claims in which:
Fig. 1 shows a block diagram of one embodiment of a peripheral nerve stimulation system 10;
Fig. 2 shows a block diagram of another embodiment of a peripheral nerve stimulation system 10;
Fig. 3 shows a block diagram of some of the circuitry disposed within one embodiment of EPG 12;
Fig. 4 shows another embodiment of EPG 12 operably connected to EPG strain relief extension 33;
Fig. 5 shows one embodiment of functional block diagrams for CP 14, PP 16, and EPG 12, with a focus on communications that occur between such components of system 10;
Fig. 6 shows various embodiments of medical electrical leads 18 and/or 20 that can be utilized in at least some embodiments of system 10;
Fig. 7(a) shows a side view of a human spine 42 and lumbar region 53;
Fig. 7(b) shows one embodiment of system 10, with leads 18 and 20 implanted within patient 22 near lumbar vertebrae L3, L4 and L5;
Fig 8 shows a dorsal view of lower portions of a human spine 42 encompassing most of lumbar region 53;
Fig. 9 shows left and right multifidus muscles 68 and 70 located dorsally from lumbar vertebrae L1 through L5 and spine 42;
Fig. 10 shows one embodiment of method 100 of implanting one or more leads 18 and/or 20 in a patient;
Fig. 11 shows one embodiment of needles 130 and 132 guided to target nerve locations 48, which are situated proximal from where medial and distal branches 44 and 46 bifurcate from dorsal ramus 52;
Fig. 12 shows a view of one embodiment or example of an optimal placement of lead 18 or 20 proximal from location 48, where the medial and dorsal branches 44 and 46 of the dorsal ramus nerves 52 bifurcate;
Fig. 13 shows one embodiment of a method 120 of electrically stimulating a patient using a dual electrical stimulation system 10;
Fig. 14 shows one embodiment of first and second stimulation signals provided to leads 18 and/or 20 by EPG 12;
Fig. 15 shows another embodiment of first and second stimulation signals provided to leads 18 and/or 20 by EPG 12;
Figs. 16 through 18 illustrate some aspects of dual stimulation regime mechanisms of action, spinal stability, breaking the cycle of spinal instability, chronic pain, patient inactivity, and muscle atrophy, and solutions provided by appropriate dual stimulation regime neurostimulation techniques combined with patient rehabilitation, and
Fig. 19 shows approximate locations of various peripheral nerves located along a line 72 beneath the head of patient 22.

The drawings are not necessarily to scale. Like numbers refer to like parts or steps throughout the drawings.

### Detailed Descriptions of Some Embodiments

Described herein are various embodiments of systems, devices, components and methods for treating pain and muscle disorders in a patient's body using neurostimulation techniques.

One emphasis of the present disclosure relates to various embodiments of systems, devices, components, methods and therapies directed to a dual electrical stimulation regime delivered from an external pulse generator (EPG) through percutaneous medical electrical leads to a patient's dorsal rami nerves for the purpose of both rehabilitating and strengthening the patient's multifidus muscles and reducing the patient's lower back pain. Other applications and embodiments for stimulating other nerves and muscles are contemplated, however, such as those employing fully implantable IPGs and/or leads, or those which stimulate muscles and sensory nerves other than the multifidus muscles and the dorsal rami nerves, more about which is said below.

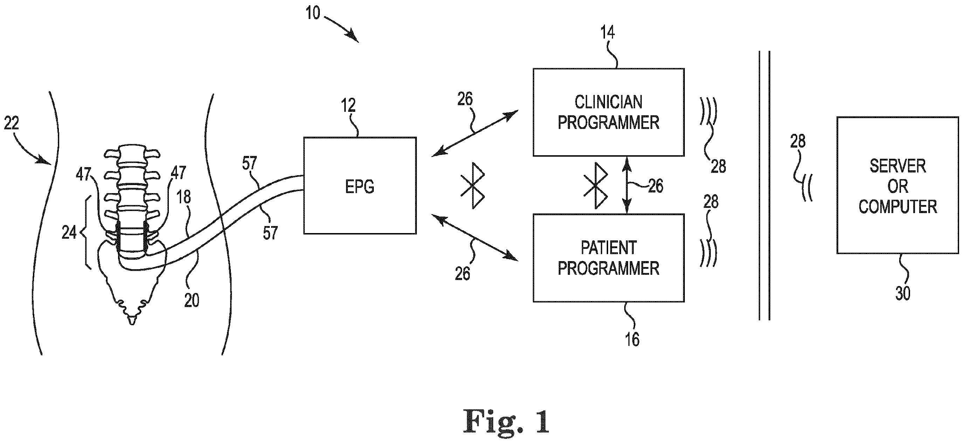

Fig. 1 shows a block diagram of one embodiment of a peripheral nerve stimulation system 10, which as shown comprises external pulse generator (EPG) 12, clinician programmer (CP) 14, patient programmer (PP) 16, first medical electrical lead 18, second medical electrical lead 20, and central server, remote computer, and/or local computer 30. Other components of system 10 are also contemplated. EPG 12 is operably connected to one, the proximal ends of two or more medical electrical leads 18 and 20, which according to one embodiment are percutaneous leads configured for placement, using a needle according to well-known practice in the medical arts, near or in proximity to a desired nerve or bundle of nerves that are then to be electrically stimulated under the control of programmable EPG 12. In other embodiments, non-percutaneous conventional medical electrical leads are also contemplated. In the embodiment shown in Fig. 1, the distal ends of leads 18 and 20 are situated in the lumbar region of patient 22 and provide electrical stimulation signals originating from EPG 12 to or near, by way of non-limiting example, dorsal rami motor and sensory nerve bundles. The electrical stimulation therapy and parameters of EPG 12 may be programmed by CP 14 under the control of a physician or other health care provider and/or may be stored and preprogrammed in a memory of EPG 12. PP 16 operates under the control of patient 22, and may be configured to permit patient 22 to turn EPG 12 on or off, to change electrical stimulation parameters (within certain limits), or to effect other changes in the operation of EPG 12. In one embodiment, CP 14 is configured to permit a physician or other health care provider to program PP 16 via wireless or other communication and connection means (e.g., Bluetooth, RF, telemetry, inductive or magnetic coupling, cable, etc.) 26. Remote or local server or computer 30 is configured to receive and/or transmit data, programming instructions, and the like from and to CP 14 and/or PP 16, as well as to process, analyze, and facilitate interpretation of such data.

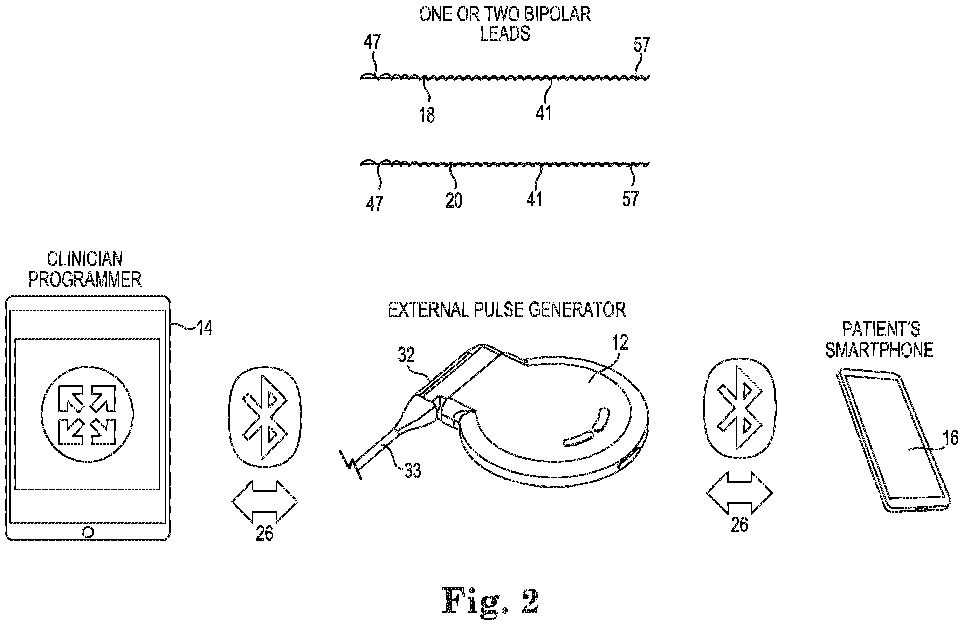

Fig. 2 shows a block diagram of another embodiment of a peripheral nerve stimulation system 10, which as shown comprises external pulse generator (EPG) 12 comprising connector block 32, which may be configured to accept the proximal ends of leads 18 and 20 therein, or to accept the proximal end of EPG strain relief extension 33 therein. Clinician programmer (CP) 14 is shown as a tablet device configured to communicate wirelessly (e.g., via Bluetooth) with EPG 12 and/or patient 22's PP 16 (which as shown in Fig. 2 is a smart phone). PP 16 is configured to permit patient 22 to activate, deactivate, program and/or adjust the electrical stimulation parameters and operation of EPG 12. EPG strain relief extension 33 provides strain relief between EPG 12 lead(s) 18 and/or 20 to minimize the possibility of lead(s) 18 and/or 20 working their way loose or otherwise moving away from their proper implanted locations within patient 22. As further shown in Fig. 2, one or two bipolar leads 18 and 20 may be employed in system 10; other numbers and types of medical electrical leads are contemplated for use in system 10, more about which is said below. Other components of system 10 are also contemplated. EPG 12 is operably connected to one, the proximal ends of two or more medical electrical leads 18 and 20. The electrical stimulation therapy and parameters of EPG 12 may be programmed by CP 14 under the control of a physician or other health care provider and/or may be stored and preprogrammed in a memory of EPG 12. PP 16 operates under the control of patient 22, and may be configured to permit patient 22 to turn EPG 12 on or off, to change electrical stimulation parameters (within certain limits), or to effect other changes in the operation of EPG 12.

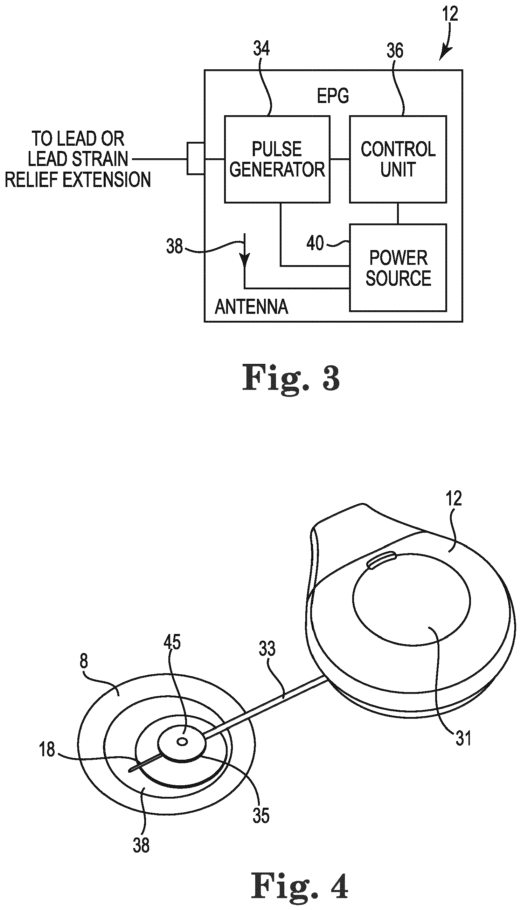

Fig. 3 shows a block diagram of some of the circuitry disposed within one embodiment of EPG 12, which as shown includes pulse generator 34, control unit 36 (*e.g.,* a CPU, processor, microprocessor, etc.), power source 40 (*e.g.,* a primary battery or batteries, a secondary or rechargeable battery or batteries, one or more capacitors, etc.), antenna 38 (for receiving and/or transmitting data, information, and/or instructions to external devices such as PP 14 and CP 18). Lead(s) 18 or 20 and/or EPG strain relief extension 33 can be operably attached to EPG 12 via EPG connector block 32.

Fig. 4 shows another embodiment of EPG 12 operably connected to EPG strain relief extension 33, the distal end of which is operably connected to strain relief extension lead connector 45. Strain relief extension lead connector 45 clips into or is otherwise affixed to EPG strain relief extension cradle 35, the underside of which is attached to adhesive pad or patch 43. (In one embodiment, adhesive pad or patch 43 is formed of TEGADERM manufactured by 3M of St. Paul, Minnesota.) Lead(s) 18 and/or 20 are then operably connected to the distal end of strain relief extension lead connector 45. Connector (or "patient cable") 45 and the distal end thereof can be operably attached to EPG 12 and/or lead 18 by an attachment or compression holding mechanism that also is configured to pierce the insulation of the connector and/or lead. Other means of attaching connector or patient cable 45 to EPG 12 and/or lead 18 and/or are also contemplated, such as set screws, conventional EPG or IPG connector blocks as are well known in the art, magnetic means, heat shrink tubing, electrically conductive or other adhesives or epoxies, and so on. Patch or pad 43 is configured for removable attachment to patient's skin 8. EPG Access cover 31 permits a technician or health care provider to, by way of non-limiting example, swap out batteries, or repair, maintain, or change other components disposed inside EPG 12. Note that some embodiments of EPG are configured to operate in conjunction with a single lead 18, dual leads 18 and 20, or more than two leads (*e.g.*, 3 leads, 4 leads, etc.).

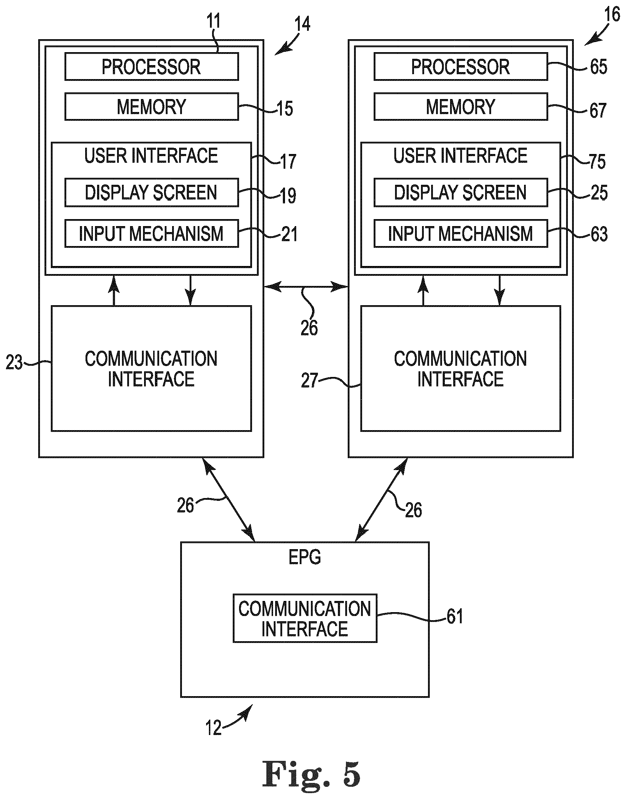

Fig. 5 shows one embodiment of block diagrams for CP 14, PP 16, and EPG 12, with a focus on communications that occur between such components of system 10. As shown in Fig. 5, Bluetooth or other communication means 26 are employed for communication between system components 14, 16, and 12. CP 14 includes processor or CPU 11, memory 15, which among other things stores programming instructions and control instructions to operate and control EPG 12, and user interface 17, which can include a screen 19 and an input mechanism 21 (*e.g.*, keypad, microphone, buttons, etc.). Communication interface 59 is configured to permit wireless or wired communications with EPG 12 and/or PP 16. Communication interface 61 is configured to communicate wirelessly or in a wired manner with CP 14 and/or PP 16. PP 16 comprises display screen 25, communication interface 27, and input mechanism 63.

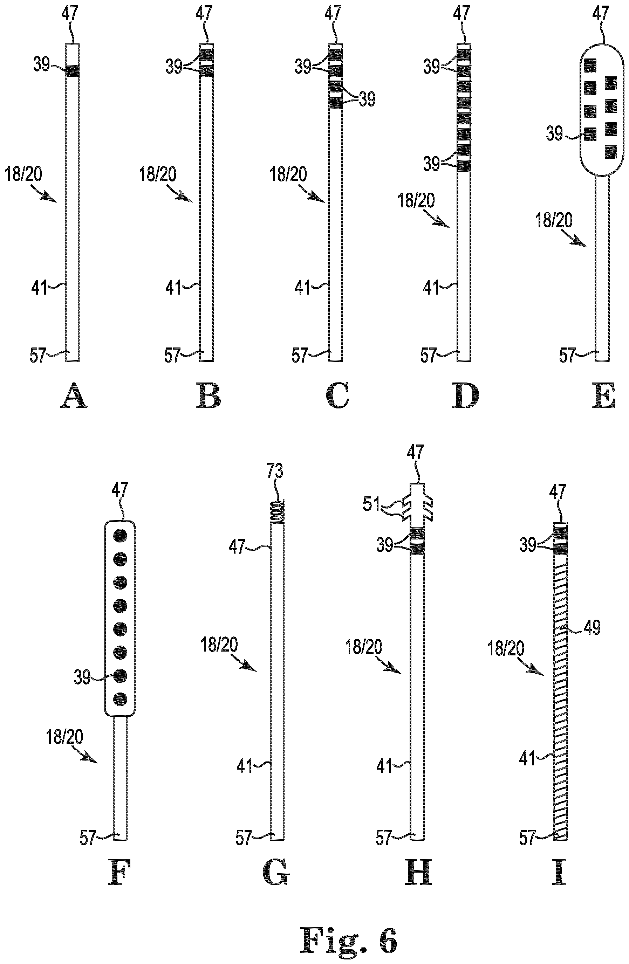

Fig. 6 shows various embodiments of medical electrical leads 18 and/or 20 that can be utilized in at least some embodiments of system 10. The dimensions of leads 18/20 shown in Fig. 6 are merely illustrative, and are not intended to be limiting. The various embodiments of medical electrical leads 18 and/or 20 shown in Fig. 6 include the following:
- Lead A - a unipolar lead with a lead body 41 and a single electrode 39 disposed near its distal end 47;
- Lead B - a bipolar lead with a lead body 41 and two electrodes 39 disposed near its distal end 47;
- Lead C - a quadripolar lead with a lead body 41 and four electrodes 39 disposed near its distal end 47;
- Lead D - an octopolar lead with a lead body 41 and eight electrodes 39 disposed near its distal end 47;
- Lead E - a paddle lead with a lead body 41 and a plurality of paddle electrodes 39 disposed in two columns;
- Lead F - a paddle lead with a plurality of electrodes 38 disposed in a single column;
- Lead G an active fixation lead with a helically wound wire coil 49 disposed at its distal end 47, where coil 49 serves both as a fixation device 49 and an electrode 39;
- Lead H - a tined lead with one or more flexible or deformable tines 57 disposed near its distal end 47; and
- Lead I - a bipolar lead with a lead body 41 and two electrodes 39 disposed near its distal end 47. In some embodiments, cuff electrode leads may also be employed, as is known in the neurostimulation arts.

Other non-limiting examples of medical electrical leads 18 and/or 20 suitable for use in some embodiments include leads used in conjunction with one or more ground electrodes, leads having arrays of cathodes employed in various configurations respecting corresponding anodes (all serving as electrodes 39), wire electrodes 39, hook-shaped electrodes 39, and barb-shaped electrodes 39. In a case where a lead 18 or 20 comprises three or more electrodes 39, EPG 12 can be configured to controllably switch and control one or more specific pairs or other groupings of electrodes 39 to which electrical stimulation is delivered in various combinations as anodes and/or cathodes. Likewise, pairs or other groups of electrodes 39 in different leads 18 and 20 (by way of non-limiting example) can be controllably switched or controlled so that the electrical fields emitted by electrodes 39 extend at least some distance between the different leads 18 and 20. In such a manner, optimum electrode pairings or groupings tailored to the specific patient 22, lead(s) placement, nerve location, etc., can be achieved to deliver the best therapy to patient 22.

In some embodiments, each of leads 18 and 20 comprises at least one cathode (electrode 39) that can be placed near a portion of the dorsal ramus nerve that contains motor and sensory components, allowing both pain blocking and muscle stimulation. Alternatively, more than one cathode (electrode 39) can be utilized, placing one cathode near a sensory component and one cathode near a motor component of the dorsal ramus nerve. Pain reduction stimulation signals are then delivered via the sensory-placed electrode, while motor stimulation of the multifidus muscle is effected via the other cathode. Both such electrodes can be mounted on a single lead, or on separate leads. As one of the electrodes is being used as a cathode for stimulation, the other electrode can be used as an anode for a return path to complete the electrical circuit. Alternatively, both stimulation electrodes could utilize a(n) additional electrode(s) as the anode. This anode could be on the one or more leads described above, a separate lead, or an external ground pad or other grounding device.

The lead examples and embodiments shown in Fig. 6 are not intended to be limiting or exhaustive, but are merely illustrative of different types of leads that can be employed in system 10. Other types and configurations of medical electrical leads other than those shown in Fig. 6 are contemplated, including various permutations and combinations of the different lead elements and components shown in Fig. 6.

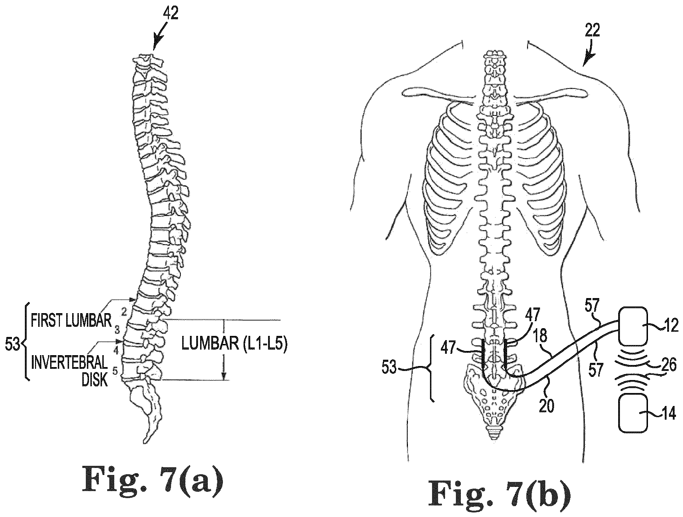

Fig. 7(a) shows a side view of a human spine 42 and lumbar region 53 comprising lumbar vertebrae L1 through L5. In one embodiment, dorsal rami nerve bundles located near or in proximity to lumbar vertebrae L3, L4 and/or L5 have been discovered to be good locations for delivering efficacious muscle rehabilitation/strengthening and lower back pain therapies to a patient 22. Fig. 7(b) shows one embodiment of system 10, with leads 18 and 20 implanted within patient 22 near lumbar vertebrae L3, L4 and L5 so as to deliver the dual stimulation muscle rehabilitation and pain relief regime described above. EPG 12 is operably connected to leads 18 and 20, which in one embodiment have been percutaneously implanted within patient 22. As described above, clinician programmer 14 is employed to set up and control the electrical stimulation parameters of EPG 12.

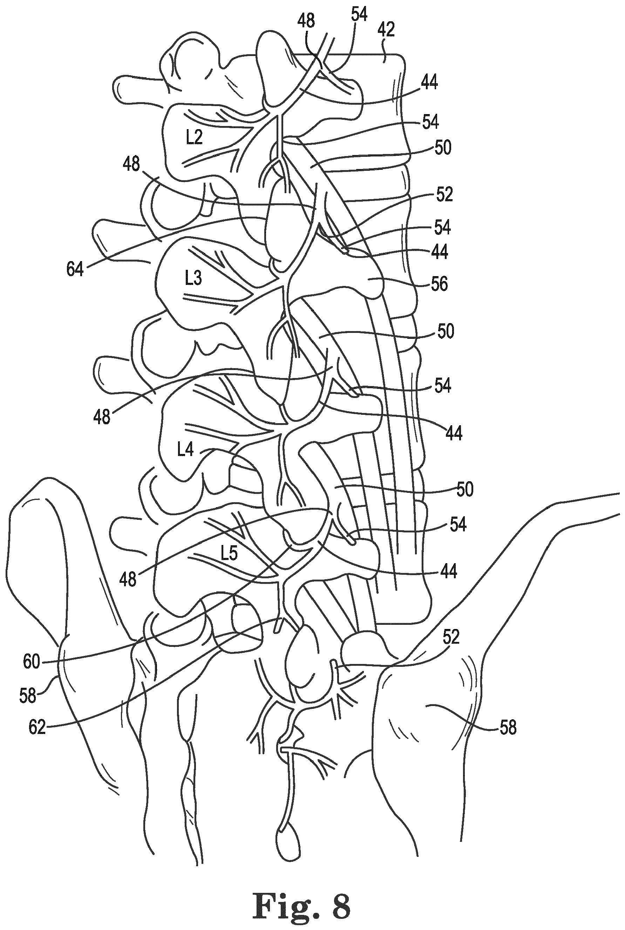

Fig 8 shows a dorsal view of lower portions of a human spine 42 encompassing most of lumbar region 53. Shown in Fig. 8 are lumbar vertebrae L2, L3, L4 and L5, and dorsal primary rami nerves 52 associated therewith and/or in proximity thereto. Also shown are medial branches of dorsal ramus nerves 44, distal branches of dorsal ramus nerves 46, and the locations 48 where medial branches of dorsal ramus nerves 44 and distal branches of dorsal ramus nerves 46 split from dorsal primary nerves 52. See also iliac crest 58, interior articular branch 60, superior articular branch 62, facet joint 64, and intermediate branch plexus 66.

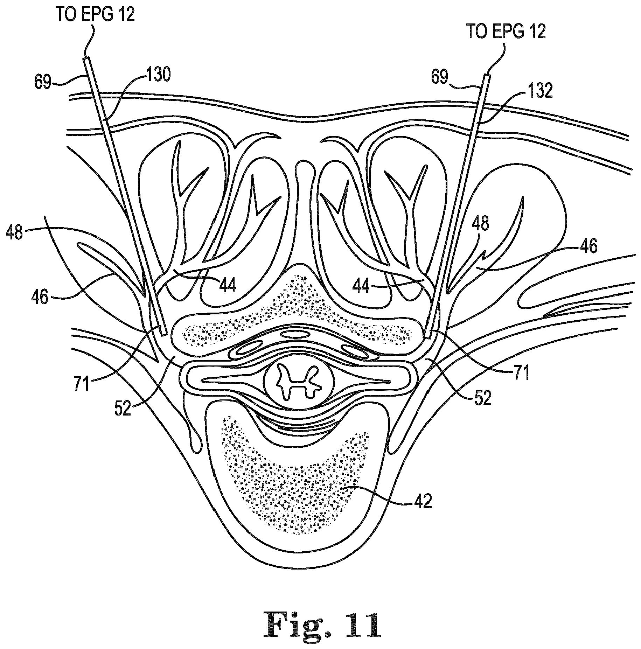

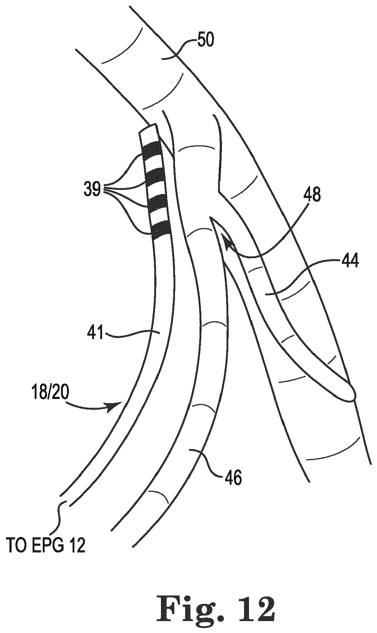

For purposes of rehabilitating a multifidus muscle and also of suppressing or reducing lower back pain using the dual stimulation regime described above, it has been discovered that in some embodiments one or more stimulation electrodes 39 are most beneficially positioned such that the one or more electrodes 39 are positioned proximal or just proximal from the bifurcation of medial and distal branches of the dorsal rami nerves at locations 49 (*i.e.,* proximal from locations 48 shown in Fig. 8, and as further shown in Figs. 11 and 12). Consistent with the improved efficacy of some embodiments of the dual electrical stimulation therapy regimes described and disclosed herein, and in accordance with our research and investigations, the dorsal primary nerves 52 are believed to contain greater numbers or proportions of mixtures or bundles of intertwined and/or interpositioned combinations of motor and sensory nerves than are to be found separately in either the medial branches of the dorsal ramus nerves 44, or in the distal branches of the dorsal ramus nerves 46. Indeed, our research and investigations have revealed that the medial branches of the dorsal rami nerves appear to contain principally motor nerves, while the lateral branches of the dorsal rami nerves appear to contain principally sensory nerves. Stimulating one or the other of the medial and lateral branches of the dorsal rami nerves will therefore provide different - and sometimes inadequate -- results to the patient, more about which is said below. Contrariwise, stimulating at or near locations 49 can provide improved results to the patient, as both motor and sensory nerves are being stimulated, which helps "break the cycle," as discussed in detail below. Consequently, in some embodiments, delivery of the dual electrical stimulation therapy regimes described and disclosed herein to locations 49 (see, e.g., Figs. 11 and 12) can provide improved therapeutic results relative to delivery elsewhere along the dorsal rami nerves or their branches. Note that in Figs. 11 and 12 the intermediate branches of the dorsal rami nerves are not shown to avoid clutter and improve illustrative clarity.

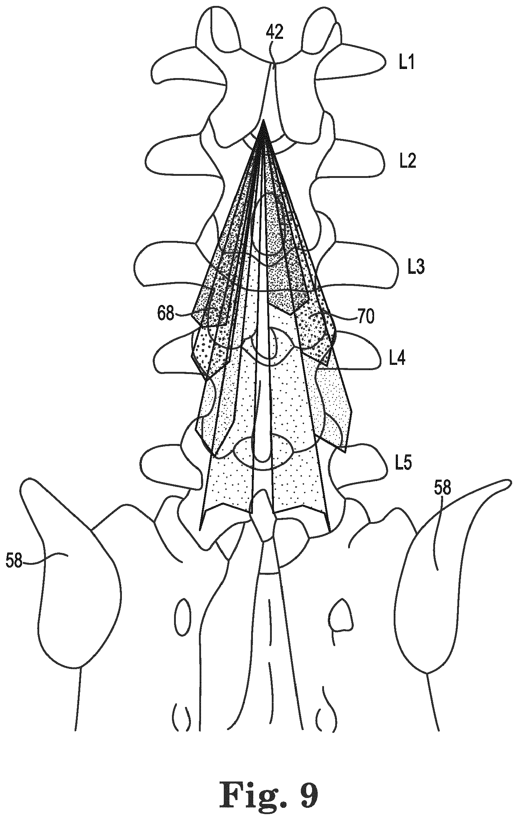

Nevertheless, and depending upon where electrodes 39 are positioned and programmed for stimulation, other locations close to or adjoining one or more of nerves 52, 44 and 46 may also be employed beneficially and efficaciously to rehabilitate or strengthen a multifidus muscle and suppress or reduced lower back pain. As shown in Fig. 9, left and right multifidus muscles 68 and 70 are located dorsally from lumbar vertebrae L1 through L5 and spine 42, but in relatively close proximity to dorsal rami nerves 52 (which in accordance with one embodiment are electrically stimulated as described above). In one embodiment, the pain treated or reduced by the second stimulation signals is non-specific chronic low back pain, or NSCLBP, heretofore a difficult and refractory condition to treat effectively.

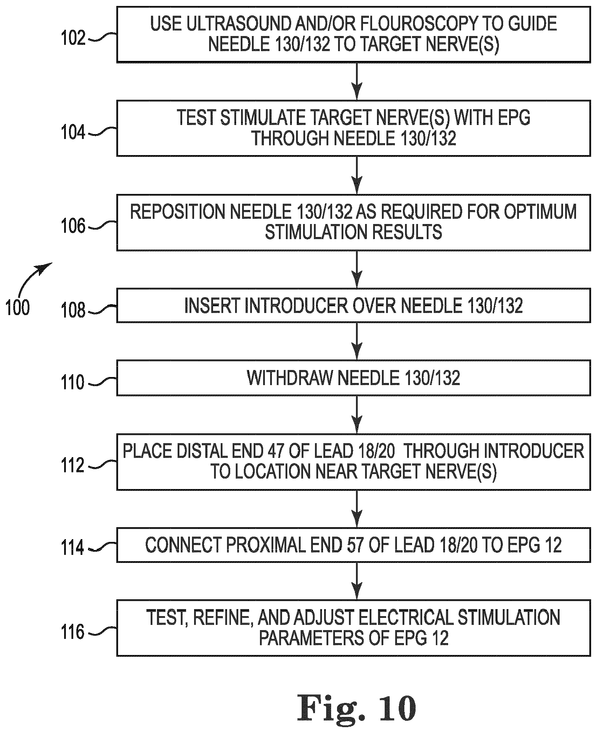

Fig. 10 shows one embodiment of method 100 of implanting one or more leads 18 and/or 20 in a patient for the purpose of simultaneously or sequentially rehabilitating multifidus muscles and reducing lower back pain. In step 102, ultrasound, fluoroscopic, MRI, PET scan, and/or CT scan techniques, or any other suitable imaging techniques, are employed to guide a test stimulation needle(s) 130 and/or 132 (see Fig. 11) to appropriate locations near one or more peripheral target nerves (*e.g*., dorsal rami nerve bundles comprising both motor and sensory nerves). By way of non-limiting example, and as shown in Fig. 11, in one embodiment, needle(s) 130 and/or 132 is guided to locations 48, which as described above are situated proximal from where medial branch of dorsal ramus and distal branches 44 and 46 of dorsal rami 52 bifurcate.

Once one or both needle(s) 130 and/or 132 have been guided to a desired location near the one or more mixed target nerves of interest, at step 104 the target nerve(s) are electrically stimulated by operably attaching the proximal ends of needles 130 and 32 to EPG 12 and activating a desired output stimulation pattern or regime for delivery to needles 130 and/or 132. Different stimulation parameters can be tested at this time by varying any one or more of the voltage, current, frequency, pulse width, amplitude, overlap, interleaving, and separate delivery of the first and second stimulation signals, as well as other electrical stimulation parameters.

In addition to experimenting with different stimulation parameters, needles 130 and/or 132 can be repositioned or their locations changed as required or desired at step 106 so that optimum stimulation results are obtained (*e.g*., maximum, sufficient, or acceptable multifidus muscle movement in response to the first signals, and a reduction, lowering, blocking or paresthesia as regards pain in the lower back in response to the second signal). Once step 106 has been completed, at step 108 an introducer is inserted over each needle, and at step 110 needle(s) 130 and/or 132 are withdrawn from the patient. Distal ends 47 of lead(s) 18 and/or 20 are then inserted through the introducers to their respective target nerve locations at step 112. Alternatively, needles 130 and 132 are hollow needles having inner diameters sufficiently large (e.g., 2mm or more) to accept therein percutaneous leads 18 and 20 having diameters less than the inner diameters of needles 130 and 132. Other techniques for implanting percutaneous leads 18 and 20 near dorsal rami 52 are also contemplated.

At step 114, the proximal ends of leads 18 and/or 20 are operably connected to EPG 12. Further refinement and adjustment of electrical stimulation and EPG programming instructions may then be carried out at step 116. Fig. 12 shows another view of one embodiment or example of an optimal placement of lead 18 or 20 proximal from location 48 near location 49 located on primary dorsal ramus nerve 52, location 48 being where the medial and dorsal branches 44 and 46 of the dorsal ramus nerves 52 bifurcate from one another.

As an example, patient 22 with chronic lower back pain is implanted with a lead or leads 18 and/Or 20 to be situated near the dorsal rami for blocking both pain and stimulating the stabilizing muscles 68 and 70 of spine 42. An appropriate nerve target is identified using a percutaneous needle stick and demonstrating activation of the target muscle as viewed using an ultrasound apparatus. Once the target nerve and location have been established, percutaneous lead(s) 18 and/or 20 are inserted using standard techniques. Lead(s) 18 and/or 20 are operably connected to EPG 12. System 10 and EPG 12 are then programmed using a clinician programmer app in CP 14 to determine appropriate stimulation parameters (e.g., amplitude, frequency, pulse width, time between delivery of the first and second signals, etc.) for patient 22.

In addition, an MRI can be used to image one or more multifidus muscles in the patient to assess the strength or degree of atrophy of the multifidus muscles 68 and 70 before the leads 18 and/or 20 are implanted in patient 22. An MRI may also be used to image one or more multifidus muscles 68 and 70 in patient 22 after therapy has been delivered to patient 22 by the first and second stimulation signals 140 and 142, and after the leads 18 and/or 20 have been implanted in patient 22.

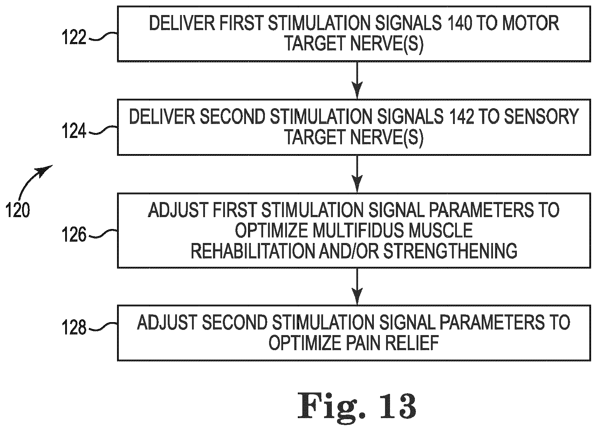

Referring now to Fig. 13, there is shown one embodiment of a method 120 of electrically stimulating a patient using a dual electrical stimulation system 10 as described herein. At step 122, first stimulation signals are delivered to motor target nerves. At step 124, second stimulation signals are delivered to sensory target nerves. At step 126, first stimulation signal parameters are adjusted to optimize multifidus muscle rehabilitation and/or strengthening in patient 22. At step 128, second stimulation signal parameters are adjusted to optimize pain relief for patient 22.

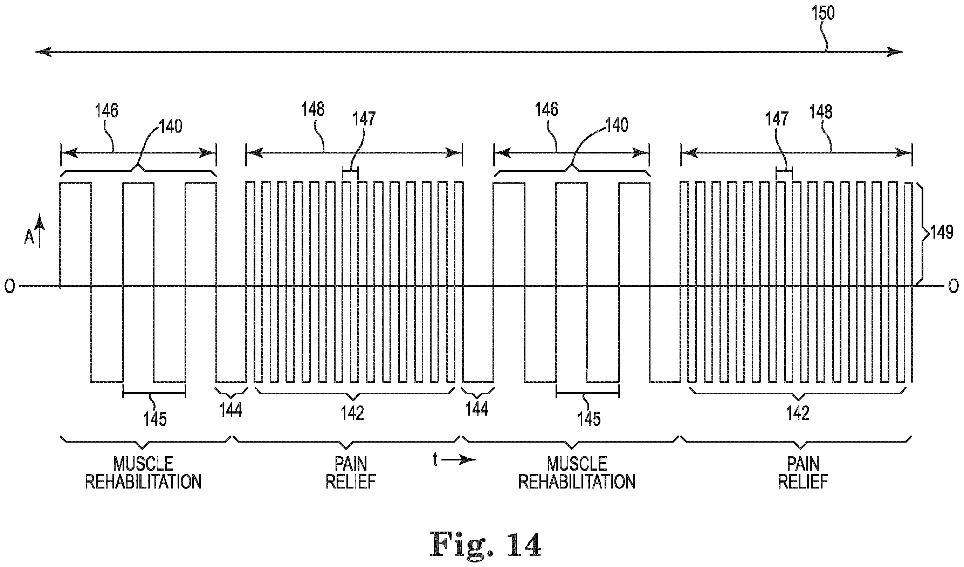

Fig. 14 shows one embodiment of first and second stimulation signals provided to leads 18 and/or 20 by EPG 12. Note that EPG 12 can be programmed to provide a wide variety of stimulation parameters for the first stimulation signal 140 and the second stimulation signal 142. Many different waveform parameters for each of the first and second stimulation signals 140 and 142 may be selected, as discussed in further detail below.

In all such potential combinations of waveform parameters in the various embodiments, however: (a) the first stimulation signals have a first range of frequencies; (b) the second stimulation signals have a second range of frequencies; (c) the average or median of the first range of frequencies is lower than the average or median of the second range of frequencies; (d) the first and second stimulation signals are delivered to the same or nearby one or more target peripheral nerves; (e) the first and second stimulation signals may be delivered at the same time, overlap one another, and/or be delivered separately but sequentially through the same, separate or multiple lead(s).

To avoid potential confusion, note that the terms "first stimulation signal" and "second stimulation signal" are not intended to mean, for example, limiting delivery of the first stimulation signal to be first in time with respect to the second stimulation signal; either signal can be delivered first, second or at some other point in time. Additionally, the generation and delivery of signals that could be classified according to their frequency as first or second stimulation signals, but that are modified or different in some respect with respect thereto (*e.g*., frequency, pulse width, amplitude, phase, etc.), which have been or will be generated and/or delivered at some previous or later point(s) in time, are also contemplated. Thus, the generation and delivery of more than first and signal stimulation signals is contemplated.

Continuing to refer to Fig. 14, there is shown one possible programming configuration. Each portion of the dual stimulation regime therapy session can be independently programmed for multiple parameters, including amplitude, frequency, pulse width, and duration. The delay (if any) between each portion can also be programmed, as can the number of sessions that occur each time the program runs. For example, EPG 12 can deliver one portion of stimulation and therapy at 10 Hz intended for muscle stimulation, programmed for a 30-minute duration, followed by a second portion of stimulation and therapy at 100 Hz for one hour with a programmed 10-minute delay between first and second stimulation signal delivery sessions. This pattern could be repeated indefinitely or terminate after a programmed number of sessions have been completed. System 10 is not limited to two different fixed stimulation and therapy stimulation regimes 140 and 142; one or both of the first and second stimulation signals 140 and 142 can be changed or modified over time, according to desired changes in stimulation patterns and therapies. Alternatively, the two different waveforms shown in Fig. 14 can be delivered simultaneously through two or more separate electrodes (or through the same electrodes as a mixed signal).

In one embodiment, and as discussed above, the first stimulation signals are configured to stimulate one or more motor nerves in the one or more bundles of target peripheral nerves to disrupt arthrogenic inhibition of the one or more muscles and to rehabilitate or strengthen the one or more muscles, and the second range of stimulation signals is configured to stimulate one or more sensory nerves in the one or more bundles of target peripheral nerves to engage gate mechanisms associated therewith thereby to reduce the pain sensed by the patient.

Continuing to refer to the example first and second stimulation signals of Fig. 14, it will be seen that first stimulation signal 140 is delivered over a time duration of 146, and second stimulation signal 142 is delivered over a time duration of 148. A time interval between the first and second stimulation signals, if they do not overlap, is denoted by time period 144. In the embodiment shown in Fig. 14, first stimulation signal 140 is characterized by a signal having a time period or pulse width denoted by 145, which is inversely related to its frequency. Likewise, in the embodiment shown in Fig. 14, second stimulation signal 142 is characterized by a signal having a time period denoted by 147 (which in this case is twice the pulse width, and which is also inversely related to the frequency of signal 140). Consistent with characteristic (c) described two paragraphs above, time period 145 is greater than time period 147, and therefore the frequency of the first stimulation signal is lower than the frequency of the second stimulation signal. Note that each of the first and second stimulation signals can have a range of frequencies associated therewith, and are not limited to single- or mono-frequency signals.

Also note that in Figure 14 amplitude 145 is associated with the first and second stimulation signals. In Fig. 14, the first and second stimulation signals are shown as having the same amplitude. In other embodiments, however, the amplitudes of the first and second stimulation signals differ, and the amplitude of the first or second stimulation signal itself may be varied.

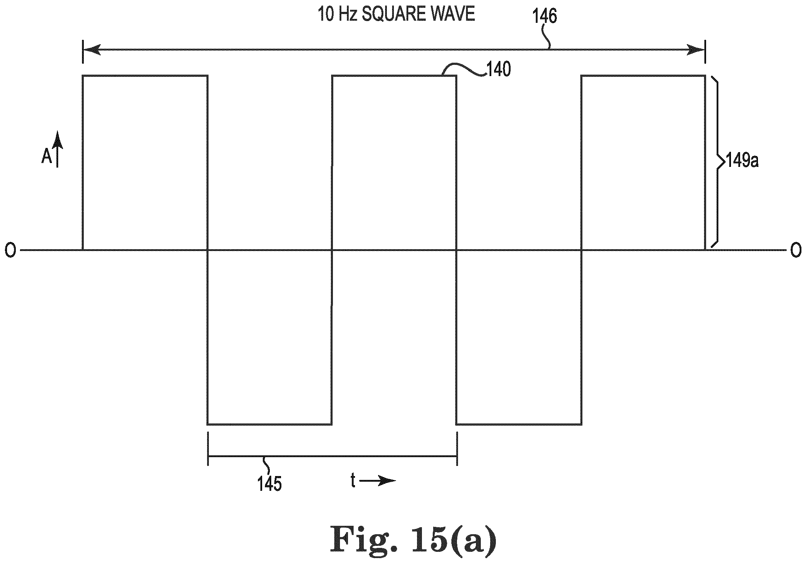

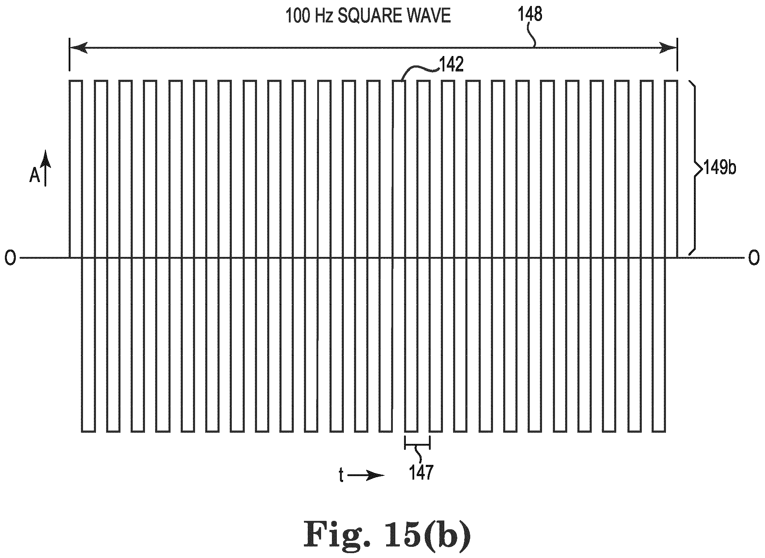

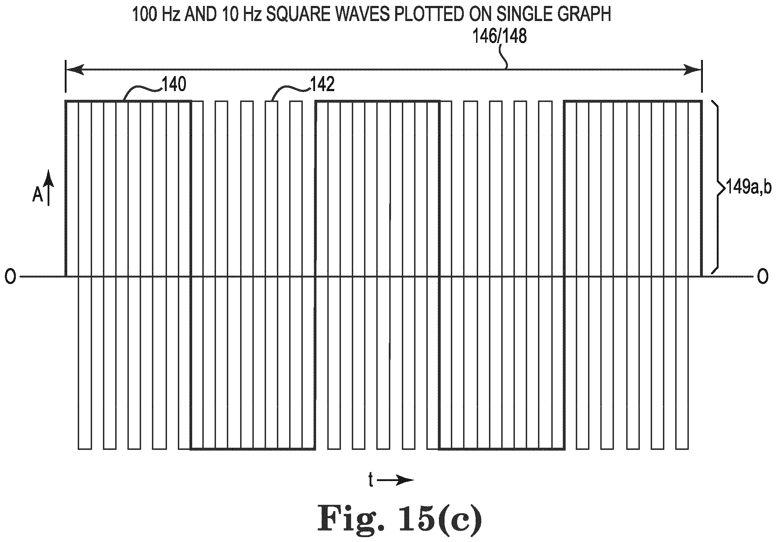

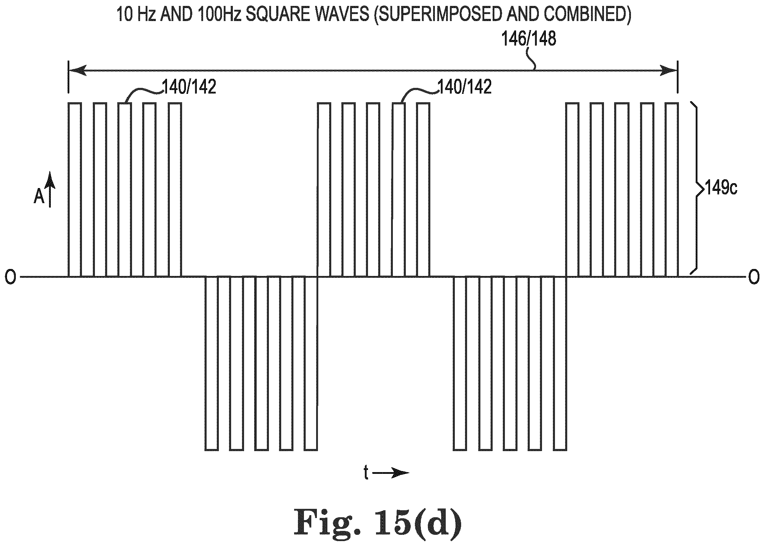

Referring now to Figs. 15(a) through 15(d), there are shown aspects of another embodiment of a composite or combined stimulation signal 140/142, which in this non-limiting example is a signal comprising a first stimulation signal having a frequency of 10 Hz (square wave signal 140 shown in Fig. 15(a)), and a second stimulation signal having a frequency of 100 Hz (square wave signal 142 shown in Fig. 15(b)). Fig. 15(c) shows first and second signals 140 and 142 plotted on the same graph. Two different signals 140 and 142 have different frequencies (10 Hz and 100 Hz, respectively), and are combined together for simultaneous (and/or overlapping) delivery to leads 18 and/or 20 as combined dual therapy signal 140/142 shown in Fig. 15(d). The combined stimulation signal embodiment shown in Fig. 15(d) illustrates one of many embodiments where first and second stimulation signals can be generated and delivered simultaneously, or can overlap with one another.

In still further embodiments, stimulation signals can be generated and delivered that comprise more than first and second stimulation signals, such as third, fourth, fifth, sixth and/or more stimulation signals, where each such combined and/or overlapping stimulation signal is characterized by a different combination or modification of stimulation parameters (*e.g*., frequency, pulse width, amplitude, phase, etc.). For example, a second pain stimulation signal having a first set of stimulation parameters associated therewith can be generated and delivered, followed by the generation and delivery of a first muscle stimulation signal having a second set of stimulation parameters associated therewith, followed by the generation and delivery of a second pain stimulation signal having a third set of stimulation parameters associated therewith, followed by the generation and delivery of a first muscle stimulation signal having a fourth set of stimulation parameters associated therewith, and so on. Pain stimulation signals can follow one after the other, and likewise muscle stimulation signals can follow one after the other. Single or multiple pain and muscle stimulation signals can be provided in any order or sequence that provides beneficial results to the patient.

In some embodiments, one or more stimulation parameters of the first muscle stimulation signals comprise one or more of: (a) frequencies ranging between about 2 Hz and about 100 Hz; (b) frequencies ranging between about 2 Hz and about 75 Hz; (c) frequencies ranging between about 4 Hz and about 50 Hz; (d) frequencies ranging between about 5 Hz and about 25 Hz; (e) frequencies ranging between about 7 Hz and about 100 Hz; (f) voltage ranging between about 0.1 mV and about 30 V; (g) current ranging between about 0.1 mA and about 30 mA; pulse width ranging between about 20 µsec and about 1000 µsec. The first stimulation signal may also be provided as a constant voltage signal or a constant current signal.

In various embodiments, one or more stimulation parameters of the second pain reduction stimulation signals comprise one or more of: (a) frequencies ranging between about 100 Hz and about 10,000 Hz; (b) frequencies ranging between about 100 Hz and about 5,000 Hz; (c) frequencies ranging between about 100 Hz and about 2,000 Hz; (d) frequencies ranging between about 100 Hz and about 1,000 Hz; (e) frequencies ranging between about 200 Hz and about 750 Hz; (f) voltage ranging between about 0.1 mV and about 30 V; (g) current ranging between about 0.1 mA and about 30 mA; pulse width ranging between about 20 µ msec and about 1,000µsec. The first and second stimulation signals may also be provided as constant voltage signals, constant current signals, triangular signals, biphasic signals, triphasic signals, chirp or swept signals, standard rectangular pulse signals, burst signals, and so on. Tapering of signals using, for example, Hanning, Hamming, and/or Blackman windowing techniques, may also be employed.

In selected embodiments, the first stimulation signals are one or more of: (a) interleaved or alternate with the second and/or other stimulation signals; (b) overlap with the second and/or other stimulation signals; (c) are at least partially superimposed upon and delivered simultaneously with the second and/or other stimulation signals; (d) delivered to the one or more target nerves at different times than when the second and/or other stimulation signals are delivered to the one or more target nerves; and/or (e) delivered to the one or more target nerves for periods of time ranging between about 60 seconds and about 180 minutes.

In further embodiments, the second stimulation and/or other signals are one or more of: (a) delivered to the one or more target nerves for periods of time ranging between about 60 seconds and about 180 minutes.

In various embodiments, the first and/or other stimulation signals are delivered to the one or more target nerves in bursts ranging between about 20 seconds and about 60 seconds in duration, and/or the second and/or other stimulation signals are delivered to the one or more bundles of target nerves in bursts ranging between about 20 seconds and about 120 seconds in duration. Such bursts can be delivered sequentially.

In selected embodiments, delivery of the first and/or other stimulation signals is separated from delivery of the second and/or other stimulation signals by a period of time ranging between: (a) about 0 seconds and about 60 seconds; (a) about 2 minutes and about 120 minutes; (a) about 1 hours and about 3 hours.

Some illustrative embodiments of generating and delivering first pain therapy stimulation signals and second muscle therapy stimulation signals are now described, where leads 18 and/or 20 have been deployed to appropriate locations near the dorsal rami nerves, and where lower back pain and multifidus muscle rehabilitation and/or strengthening therapies (e.g., the second and first stimulation signals) are delivered to patient 22.

In one embodiment, a first muscle therapy session is delivered to the patient using a first muscle rehabilitation therapy stimulation signal having a frequency of about 10-12 Hz for about 2 hours. In a subsequent pain therapy session, a second pain therapy stimulation signal having a frequency of about 100 Hz is delivered to the patient for about 30 minutes. A second muscle rehabilitation therapy session is then delivered to the patient using the first muscle rehabilitation stimulation signal having a frequency of about 10-12 Hz for about 1 hour. A break in the delivery of the first and second stimulation signals (e.g., 2- to 4-hours) is then taken, followed by repeating the first muscle rehabilitation therapy session, the second paint therapy session, and the second muscle rehabilitation therapy session. During an average waking day for a patient, two or three of the foregoing muscle rehabilitation and pain therapy sessions can be delivered to the patient. Such therapy is typically delivered over a 45-60 day time period (or longer)

Therapy sessions can be adjusted or modified as required over the multi-day or multi-month time period over which the first and second stimulation signals are delivered to the patient. For example, the stimulation parameters of pain and/or muscle rehabilitation therapy sessions can be changed or modified as a day, or the multi-day or multi-month time period, progresses. Pain therapy sessions can be shortened as the patient's pain is reduced and the multifidus muscles become stronger. In some embodiments, the initial focus on treatment and therapy is to reduce the patient's lower back pain first so that the patient can resume or increase physical activity, which in turn permits subsequent therapy to focus increasingly on multifidus muscle strengthening, thereby breaking the cycle (as described in further detail below). Many different modifications, combinations, and permutations of pain and muscle rehabilitation therapy sessions are contemplated, as those skilled in the art will understand after having read and understood the present specification and claims.

In another embodiment, at least some of the pain therapy sessions can include second stimulation signals having frequencies ranging between about 1,000 Hz and about 10,000 Hz. Such high frequency pain stimulation signals can provide patients with reduced-impedance signals (which in some cases can penetrate tissue and nerves deeper and further than lower-frequency signals), as well as paresthesia-free pain relief.

In still further embodiments, electrodes 39 on leads 18 and/or 20 may also be employed not only to stimulate targeted nerve bundles or nerves, but also to sense depolarization and repolarization signals originating from the targeted nerve bundles or tissue in proximity thereto. These sensed signals may in turn be employed by programming instruction loaded and circuitry disposed in EPG 12 to process the sensed signals, and then determine whether or not the stimulation parameters of the first and/or second stimulation signals should be adjusted, thereby forming a feedback control loop for peripheral nerve stimulation.

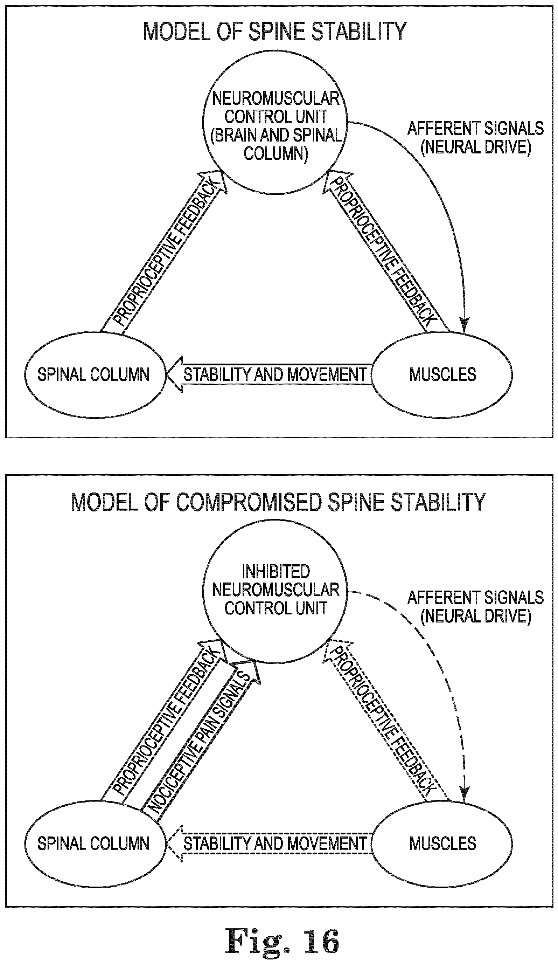

Referring now to Figs. 16 through 18, there are illustrated some aspects of dual stimulation regime mechanisms of action, spinal stability, breaking the cycle of spinal instability, chronic pain, patient inactivity, and muscle atrophy, and solutions provided by appropriate dual stimulation regime neurostimulation techniques combined with patient rehabilitation.

The top portion of Fig. 16 illustrates a model of normal spinal stability in a patient 22 who is experiencing no or few symptoms of spinal instability such as scoliosis, and no or little lower back pain. As shown in the top portion of Fig. 16, the spinal column, back muscles (including multifidus muscles), and nerves associated with neuromuscular control and function are in balance with one another.

The bottom portion of Fig. 16 illustrates a model of abnormal or compromised spinal stability in a patient 22 who is experiencing symptoms of spinal instability such as scoliosis, and uncomfortable if not worse lower back pain. As shown in the bottom portion of Fig. 16, the spinal column, back muscles (including multifidus muscles), and nerves associated with neuromuscular control and function are not in balance with one another, and patient 22 suffers spinal instability and lower back pain as a result.

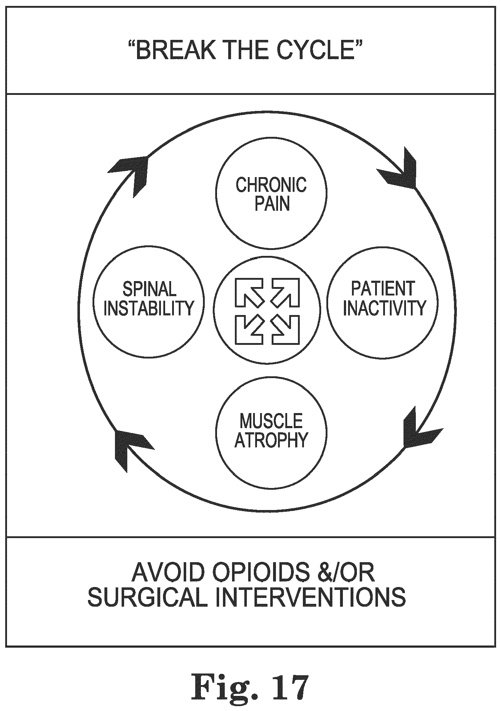

Fig. 17 illustrates the feedback cycle or loop in which many patients who suffer from spinal instability and lower back pain find themselves, namely a cycle in which the patient has spinal instability, lower back or other pain results, the patient becomes inactive because activity and exercise exacerbate the effects of spinal instability and lower back pain, and finally the resultant atrophy of the multifidus (and sometimes other) back muscles. If the cycle is not broken, the patient may wind up using opioids for pain relief and/or require surgical intervention in a bid to restore spinal stability. The dual stimulation regime therapies described and disclosed herein are intended to break this cycle while avoiding the use of pain pharmaceuticals or drugs, and eliminating the need for surgical intervention.

Continuing to refer to Fig. 17, a patient's spine stabilization system comprises the spine, certain back muscles, and a neural control system. Arthrogenic muscle inhibition can disrupt control to key segmental stabilizing muscles of the spine, such as the lumbar multifidus muscle (LMM). Disrupted muscle control can lead to clinical instability of the spine, allowing joint overload and consequent persistent and recurrent pain. Back pain due to disrupted muscle control is associated with neuroplastic changes in the motor cortex, which can be reversed with elimination of back pain. Consequently, targeting multifidus muscle control and lower back pain using the dual electrical stimulation regimes described and disclosed herein is a new treatment option for NSCLBP.

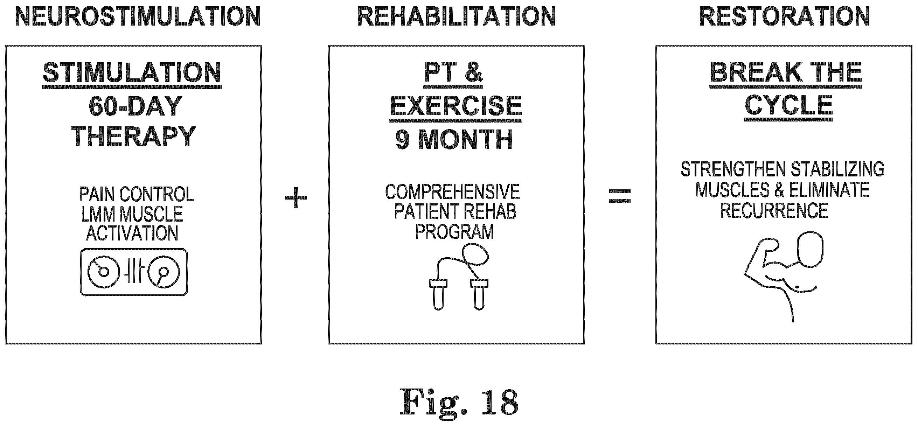

Fig. 18 is an illustrative (but not intended to be limiting) embodiment of a therapy regime that can be employed to help a patient recover spinal instability and lower back pain. First, a dual electrical stimulation regime is delivered to the patient in accordance with the descriptions and disclosures set forth herein. In the example of Fig. 18, this stimulation regime is delivered to the patient over a 60-day period. Note that other periods of time for this first period are also contemplated, including, but not limited to, about 15 days, about 20 days, about 30 days, about 45 days, about 60 days, about 70 days, about 80 days, about 90 days, and even longer periods of time. In the second step, and after the first step has been completed and restoration of the patient's spinal stability has begun and lower back pain has at least been suppressed, the patient engages in some combination of physical therapy and exercise for a period of time (e.g., about 9 months, about 3 months, about 6 months, and/or about 1 year). In the final third step, restoration of spinal stability is achieved, where the cycle is broken, the multifidus muscles have been strengthened, rehabilitated and stabilized, and lower back pain has been eliminated or substantially reduced.

Referring now to Figs. 16 through 18, Peripheral Nerve Stimulation (PNS) is thought to be one of the key elements of a mechanism of action (MOA) proposed to be responsible for modulation of central sensitization creating sustained analgesic effects among patients with chronic back pain of both nociceptive and neuropathic characteristics ("delivery of therapy"). In addition to stimulation of afferent fibers, which is believed to engage the gate mechanism directly to reduce pain signaling, stimulation of efferent fibers activates muscles and thereby is believed to generate proprioceptive afferent signals from the muscle spindles and Golgi tendon organs activated in those muscles ("stimulation"). Together, these afferent signals may help to normalize or partially reverse membrane excitability of neurons and circuits in the pain processing pathways ("normalization"). This reduction in pain signals with PNS may also disrupt the cycle of centrally mediated pain, permitting restorative levels of activity, which may further reduce pain via activity-dependent neuroplasticity even long after therapy has been delivered ("sustained normalization" and "breaking the cycle").

Some articles and technical papers describing and disclosing certain selected aspects of multifidus muscle rehabilitation and lower back pain systems, devices, methods, and therapies described and disclosed herein include the following publications: (a) Peripheral Nerve Stimulation for Chronic Low Back Pain: Prospective Case Series With 1 Year of Sustained Relief Following Short-Term Implant, Gilmore CA et al, Pain Practice 2020 Mar;20(3):310-320; (b) Gilmore CA, et al., Percutaneous Peripheral Nerve Stimulation for Chronic Low Back Pain: Prospective Case Series with 1 Year of Sustained Relief following Short-term Implant., Neuromodulation, vol. 22, issue 5, July, 2019; (c) Muscle Control for Non-specific Chronic Low Back Pain, Russo et al., Neuromodulation 2018:: vol. 21, pp. 1-9; (d) Deckers, K et al, New Therapy for Refractory Chronic Mechanical Low Back Pain-Restorative Neurostimulation to Activate the Lumbar Multifidus: One Year Results of a Prospective Multicenter Clinical Trial. Neuromodulation, 2018 Jan;21(1):48-55; (e) Gilmore, C, et al., Reduction in Opioid Consumption Reported among Chronic Low Back Pain Patients Following Percutaneous Peripheral Nerve Stimulation (PNS) of the Medial Branch Nerve for up to 60 days, ASRA Nov 2019; (f) Gilmore CA, et al., Percutaneous 60-day Peripheral Nerve Stimulation Implant Provides Sustained Relief of Chronic Pain Following Amputation: 12-month Follow-up of a Randomized, Double-Blind, Placebo-Controlled Trial, Regional Anesthesia and Pain Medicine, 2019; (g) Deyo, Low Back Pain, N Engl J Med, 2001 Vol 344, No. 5. 363-370; (h) Burton et al., European Guidelines for Prevention in Back Pain, 2004, Eur Spine J (2006) 15 (Suppl. 2): S136-S168 (i) Hestbaek, Low back pain: what is the long-term course? A review of studies of general patient populations, Eur Spine J 2003, 12: 149-165; (j) Chou, Diagnosis and Treatment of Low Back Pain: A joint clinical practice guideline from the American College of Physicians and the American Pain Society., Ann Intern Med. 2007. 147: 478-491; (k) Hall et al., The role of radiofrequency facet denervation in chronic back pain, Jacksonville Medicine, October, 1998; (I) U.S. Patent No. 4,026,301 to Friedman entitled "Apparatus and method for optimum electrode placement in the treatment of disease syndromes such as spinal curvature;" (m) U.S. Patent No. 6,505,075 to Weiner entitled "Peripheral nerve stimulation method;" (n) U.S. Patent No. 7,167,756 to Torgerson et al. entitled "Battery recharge management for an implantable medical device;" (o) U.S. Patent No. 8,606,358 to Sachs entitled "Muscle stimulator;" (p) U.S. Patent No. 8,700,177 to Strother et al. entitled "Systems and methods for providing percutaneous electrical stimulation;" (q) U.S. Patent Publication No. 2004/0122482 to Tung et al. entitled "Nerve Proximity Method and Device;" (r) U.S. Patent Publication No. 2010/0036454 to Bennett et al. entitled "Systems and methods to place one or more leads in muscle for providing electrical stimulation to treat pain," and (s) U.S. Patent Publication No. 2013/0296966 to Wongsarnpigoon et al. entitled "Systems and methods related to the treatment of back pain." Each of the foregoing publications is hereby incorporated by reference herein, each in its respective entirety pursuant to an Information Disclosure Statement filed on even date herewith containing citations or complete copies, as the case may be, of such publications.

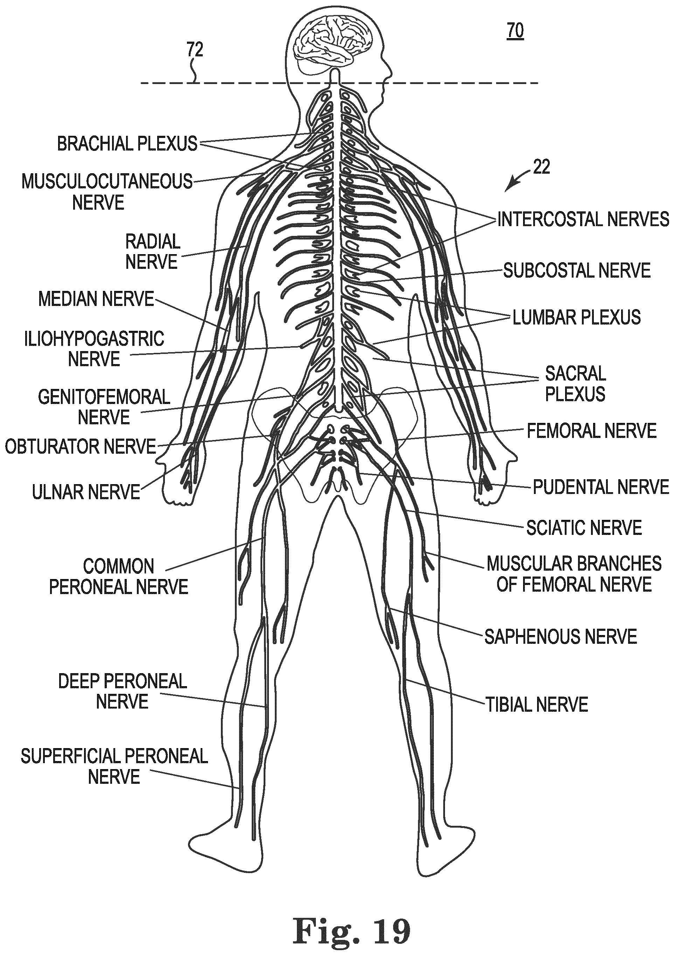

Referring now to Fig. 19, there are shown the approximate locations of various peripheral nerves located along a line 72 beneath the head of patient 22. As described above, the various embodiments of the dual electrical stimulation regime and techniques described herein find principal application in peripheral nerves and accompanying or nearby muscles that are disposed well below line 72, such as the patient's shoulder, back, knee, or ankle. Nevertheless, in some applications, such as where patient 22 suffers from atrophied neck muscles and neck pain, some embodiments can be employed in the lower, middle and/or upper regions of the neck.

Continuing to refer to Fig. 19, by way of non-limiting example, the one or more target peripheral nerves described herein as candidates for the dual electrical stimulation regime therapy described and disclosed herein may be located in or near one or more of the patient's shoulder, neck, arm, leg, knee, hip, foot, ankle, and/or other locations where target peripheral nerves reside and are in proximity to one or muscles which would benefit from electrical stimulation to rehabilitate and/or strengthen same, and where the patient would also sense that pain is reduced by electrical stimulation of such target nerves. The dual electrical stimulation systems, devices, components, methods and techniques described and disclosed herein may also be applied to relive chronic shoulder neuropathic pain and post-surgical pain.

It will now be seen that the various systems, devices, components and methods disclosed and described herein are capable of rehabilitating and strengthening atrophied muscles, and reducing or eliminating pain sensed by a patient.

What have been described above are examples and embodiments of the devices and methods described and disclosed herein. It is, of course, not possible to describe every conceivable combination of components or methodologies for purposes of describing the invention, but one of ordinary skill in the art will recognize that many further combinations and permutations of the devices and methods described and disclosed herein are possible. Accordingly, the devices and methods described and disclosed herein are intended to embrace all such alterations, modifications and variations that fall within the scope of the appended claims. In the claims, unless otherwise indicated, the article "a" is to refer to "one or more than one."

The foregoing description and disclosure outline features of several embodiments so that those skilled in the art may better understand the detailed description set forth herein. Those skilled in the art will now understand that many different permutations, combinations and variations of hearing aid 10 fall within the scope of the various embodiments. Those skilled in the art should appreciate that they may readily use the present disclosure as a basis for designing or modifying other processes and structures for carrying out the same purposes and/or achieving the same advantages of the embodiments introduced herein. Those skilled in the art should also realize that such equivalent constructions do not depart from the spirit and scope of the present disclosure, and that they may make various changes, substitutions and alterations herein without departing from the spirit and scope of the present disclosure.

After having read and understood the present specification, those skilled in the art will now understand and appreciate that the various embodiments described herein provide solutions to long-standing problems in the effective use of neurostimulation systems.

## Claims

1. A system for rehabilitating or strengthening one or more muscles in a patient, and reducing pain sensed by the patient, though electrical stimulation of one or more peripheral nerves, comprising:
one or more medical electrical leads comprising distal portions or ends comprising one or more electrodes configured for implantation adjacent to, in contact with, or in operative positional relationship to, one or more target peripheral nerves of the patient, where the one or more target peripheral nerves comprising motor and sensory nerves, and
an external pulse generator (EPG) configured for operable connection to the one or more medical electrical leads, and further being configured to deliver first stimulation signals having a first range of frequencies through the one or more electrodes of the one or more medical electrical leads to the one or more target peripheral nerves, the EPG still further being configured to deliver second stimulation signals having a second range of frequencies through the one or more electrodes of the one or more medical electrical leads to the one or more target nerves;
wherein the first range of frequencies is lower than the second range of frequencies, the first stimulation signals are configured to stimulate one or more motor nerves in the one or more target peripheral nerves to rehabilitate or strengthen the one or more muscles, the second stimulation signals are configured to stimulate one or more sensory nerves in the one or more target peripheral nerves to reduce pain sensed by the patient.

2. The system of claim 1, wherein one or more stimulation parameters of the first stimulation signals comprise one or more of: (a) frequencies ranging between about 2 Hz and about 100 Hz; (b) frequencies ranging between about 2 Hz and about 75 Hz; (c) frequencies ranging between about 4 Hz and about 50 Hz; (d) frequencies ranging between about 5 Hz and about 25 Hz; (e) frequencies ranging between about 7 Hz and about 100 Hz; (f) voltage ranging between about 0.1 mV and about 30 V; (g) current ranging between about 0.1 mA and about 30mA; pulse width ranging between about 20 msec and about 1000 msec.

3. The system of one of claims 1 and 2, wherein one or more stimulation parameters of the second stimulation signals comprise one or more of: (a) frequencies ranging between about 100 Hz and about 10,000 Hz; (b) frequencies ranging between about 100 Hz and about 5,000 Hz; (c) frequencies ranging between about 100 Hz and about 2,000 Hz; (d) frequencies ranging between about 100 Hz and about 1,000 Hz; (e) frequencies ranging between about 200 Hz and about 750 Hz; (f) voltage ranging between about 0.1 mV and about 30 V; (g) current ranging between about 0.1 mA and about 30 mA; pulse width ranging between about 0.1 µsec and about 1000 µsec.

4. The system of any one of claims 1 to 3, wherein the first stimulation signals are interleaved or alternate with the second stimulation signals.

5. The system of any one of claims 1 to 3, wherein the first stimulation signals overlap with the second stimulation signals.

6. The system of any one of claims 1 to 5, wherein the first stimulation signals are at least partially superimposed upon and delivered simultaneously with the second stimulation signals.

7. The method of any one of claims 1 to 6, wherein the first stimulation signals are delivered to the one or more target nerves at different times than when the second stimulation signals are delivered to the one or more bundles of target nerves.

8. The system of any one of claims 1 to 7, wherein the first stimulation signals are delivered to the one or more target nerves for periods of time ranging between about 60 seconds and about 180 minutes.

9. The system of any one of claims 1 to 8, wherein the second stimulation signals are delivered to the one or more target nerves for periods of time ranging between about 60 seconds and about 180 minutes.

10. The system of any one of claims 1 to 9, wherein the first stimulation signals are delivered to the one or more target nerves in bursts ranging between about 20 seconds and about 120 seconds in duration.

11. The system of any one of claims 1 to 10, wherein the second stimulation signals are delivered to the one or more target nerves in bursts ranging between about 20 seconds and about 120 seconds in duration.

12. The system of any one of claims 1 to 11, wherein delivery of the first stimulation signals is separated from delivery of the second stimulation signals by a period of time ranging between: (a) about 0 seconds and about 60 seconds; (b) about 2 minutes and about 120 minutes; (c) about 1 hour and about 3 hours.

13. The system of any one of claims 1 to 12, wherein the one or more target peripheral nerves comprise dorsal rami nerves, and preferably wherein at least one of the following holds true:
the one or more electrodes are positioned proximal to a bifurcation of medial and distal branches of the dorsal rami nerves,
the one or more muscles comprise one or more multifidus muscles,
the first stimulation signals promote rehabilitating or strengthening one or more atrophied multifidus muscles,
the pain is non-specific chronic low back pain (NSCLBP) and the second stimulation signals promote reducing non-specific chronic lower back pain.

14. The system of any one of claims 1 to13, wherein the one or more target peripheral nerves are located in or near one or more of the patient's shoulder, neck, arm, leg, knee, hip, foot, or ankle.

15. The system of any one of claims 1 to 14, wherein the one or more medical electrical leads comprise at least one of a unipolar electrode, a bipolar electrode, a ground electrode, a cathode, an anode, a coiled electrode, a cuff electrode, a wire electrode, and a hook-shaped electrode.

16. A method of operation of an external pulse generator, EPG, configured for operable connection to one or more medical electrical leads comprising distal portions or ends comprising one or more electrodes configured for implantation adjacent to, in contact with, or in operative positional relationship to, one or more target peripheral nerves of the patient, where the one or more target peripheral nerves comprising motor and sensory nerves, comprising:
deliver first stimulation signals having a first range of frequencies through the one or more electrodes of the one or more medical electrical leads to the one or more target peripheral nerves,
deliver second stimulation signals having a second range of frequencies through the one or more electrodes of the one or more medical electrical leads to the one or more target nerves;
wherein the first range of frequencies is lower than the second range of frequencies, the first stimulation signals are configured to stimulate one or more motor nerves in the one or more target peripheral nerves to rehabilitate or strengthen the one or more muscles, the second stimulation signals are configured to stimulate one or more sensory nerves in the one or more target peripheral nerves to reduce pain sensed by the patient.
